# EUROPEAN PATENT APPLICATION

(11) **EP 4 014 758 A1**
(43) Date of publication of application: **22.06.2022**
(21) Application number: 20852066.8
(22) Date of filing: 27.06.2020
(51) Int. Cl.: A23L 33/115, A61K 9/00, A61K 31/05, A61K 31/685, A61K 47/44, A61P 1/16

(54) **LIPID COMPOSITION COMPRISING ANTIOXIDANTS AND NATURAL POLYPHENOLS AS A NON-PHARMACOLOGICAL ALTERNATIVE FOR THE TREATMENT AND PREVENTION OF NON-ALCOHOLIC FATTY LIVER DISEASE (NAFLD)**

(30) Priority: 13.08.2019 US 201962885987 P
(71) Applicant: Team Foods Colombia S.A., Bogotá (CO)
(72) Inventor: COLMENARES, Daniel, Bogotá (CO); NUÑEZ, Vanessa, Bogotá (CO); CASTRO, Camilo, Bogotá (CO); BLANCO, Carla, Bogotá (CO); GOMÉZ, Luisa Fernanda, Bogotá (CO); BETANCOURT, Eddy, Bogotá (CO); ÁLVAREZ, Carlos., Bogotá (CO)
(74) Representative: Curell Suñol S.L.P.
(86) International application number: PCT/IB2020/056098
(87) International publication number: WO 2021/028737

(57) **Abstract**

The present application provides lipid-based compositions comprising bioactives of natural origin. In particular, the compositions disclosed herein comprise antioxidants, such as anthocyanins, polyphenols, such as curcuminoids, mixtures of omega-3 and omega-7 unsaturated fatty acids, phospholipids, liposoluble vitamins and medium chain triglycerides, which provide a non-pharmacological alternative for the treatment and prevention of liver diseases related to non-alcoholic fatty liver disease (NAFLD).

## Description

### BACKGROUND AND FIELD OF THE INVENTION

Non-alcoholic fatty liver disease (NAFLD) is a chronic liver disease that affects between 10 and 25% of the general population, however, this percentage increases in people suffering from obesity and diabetes, being between 57 to 74%. This increase is due to the critical role that insulin resistance and oxidative stress play in the accumulation of liver fat, and therefore in the development of the disease.

This disease is characterized by the infiltration of lipids in the liver and may progress to simple steatosis and non-alcoholic steatohepatitis (NASH), which can lead to severe liver diseases such as fibrosis, cirrhosis and hepatocellular carcinoma (HCC), as well as cardiometabolic diseases such as cardiovascular diseases and type II diabetes.

Obesity and diabetes have been found to be the major risk factors for the development of NAFLD and liver cancer. For this reason, there is a direct relationship between these diseases and the unhealthy lifestyle, characterized by sedentary lifestyle and the intake of highly caloric diets that usually include excessive consumption of saturated fat, refined carbohydrates, sucrose and fructose. Other risk factors include: (i) metabolic disorders such as lipodystrophy, hypopituitarism, disbetalipoproteinemia and Weber-Christian disease; (ii) infections such as HIV or hepatitis C; (iii) exposure to toxins such as those produced by *Amanita phalloides* or *Bacillus cereus*; (iv) prolonged use of medications such as amiodarone, diltiazem, steroids, synthetic estrogens, tamoxifen, and cocaine; (v) Severe weight loss or gastric bypass.

Now, the accumulation of liver fat is directly related to insulin resistance, which increases the lipolysis of peripheral adipose tissue, resulting in an increase in the influx of fat to the liver in the form of free fatty acids. Additionally, insulin resistance promotes the de novo synthesis of triglycerides within the liver and inhibits the oxidation of fatty acids by promoting the accumulation of triglycerides.

Therefore, a key strategy in the treatment of NAFLD is the search for mechanisms to improve insulin sensitivity. However, due to the absence of pharmaceutical products approved in the market that fulfill this function, the first recommended therapy for NAFLD is lifestyle optimization, since having a healthy diet and increasing physical activity, favors the reduction of liver fat and improves glucose control, as well as insulin sensitivity.

In this way, it is important to highlight that, although there are no drugs approved in the market to directly treat the NAFLD, there are some alternative drugs to reduce the production of sugar such as metformin, or to help remove blood sugar such as triazolidinediodines. Also, although there are some hepatoprotective agents such as: pentoxifylline, Betaine, Angiotensin II, Losartan and ursodeoxycholic acid (UDCA), these are contraindicated and, in some cases, restricted, therefore, their prescription depends on the diagnosis of each patient.

For this reason, there is currently a great interest in the development of non-pharmacological alternatives for the management of different metabolic disorders, that include some nutraceuticals, foods and / or supplements with the ability to favorably modify the course of liver diseases or to effectively impact the risk factors of this.

In this sense, in the market there are some supplements for elimination of toxins, cleaning and support to the proper functioning of the liver. These products include alternatives based on phospholipids, among which are Essentiale^{®}, Lev Forte, Optimal PC, PhosChol^{®}, Essentix^{®}, Esseliv^{®}, Essetil^{®}, and on the other hand, alternatives based on plant extracts or fungi, such as Pro-Liver & Pancreas^{™}, Liver Cleanse Plus^{®}, Liver Cleanse and Detox P960, Berberine Max, Milk Thistle-Liver Support and Liver Refresh. However, none of these alternatives provides a direct and concrete effect for the treatment of NAFLD.

On the other hand, treatment methods for liver diseases based on the use or administration of nutritional or pharmaceutical compositions have been developed.

Thus, the patent document WO2005110124 teaches nutritional compositions directed to patients with liver failure, which include an effective amount of branched-chain amino acids valine, leucine, isoleucine, or mixtures of these. These compositions may also contain a reduced amount of tyrosine, phenylalanine and tryptophan. Likewise, the composition disclosed in this document also has a source of carbohydrates and a specific proportion of omega 6 and omega 3 fatty acids.

Likewise, EP1307108B1 refers to ready-to-use compositions, useful in the treatment of patients suffering from liver disease. Said compositions have a defined ratio of macronutrients, and they contain a specific amount of free amino acids, which correspond to 25% by weight of the protein fraction.

Meanwhile, the document EP2603096 discloses compositions useful in the treatment of liver diseases, particularly NAFLD and NASH, comprising an effective amount of 27-hydroxycholesterol.

Likewise, WO2015190760A1 teaches an effective composition in the prevention and treatment of NAFLD which has as main characteristic the presence of extracts of *Aronia melanocarpa, Silybum marianum,* and *Phellinus linteus.*

In addition, documents such as CN102755350 and WO2015027463, refer to the use of oil from animal sources, which have a high content of unsaturated fatty acids such as ALA (alpha-linolenic acid), EPA (eicosapentaenoic acid), DPA (docosapentaenoic acid) and DHA (docosahexaenoic acid), in the treatment of non-alcoholic fatty liver disease.

On the other hand, WO2011097273 discloses a composition useful in the treatment of non-alcoholic fatty liver disease, which is characterized by comprising DHA and N-acetyl L-cysteine, and also by being free from EPA.

On the other hand, WO2016057915 teaches compositions for the treatment of diseases, among which is NAFLD, which comprise at least one omega-3 fatty acid (such as triglyceride or free acid) and a surfactant, so that, when the composition comes into contact with an aqueous medium, micelles are formed.

Finally, document US2010267611 discloses the use of phospholipids to minimize the accumulation of triglycerides or plasma cholesterol in patients who are at risk of developing NAFLD.

In accordance with the foregoing, it is possible to observe that, despite the fact that numerous and varied alternatives are available for the treatment of liver diseases and conditions, there is still no therapeutic alternative for the treatment and prevention of NAFLD that provides benefits on insulin sensitivity or on lipid infiltration in the liver.

Thus, the need remains to provide non-pharmacological alternatives for the prevention and treatment of NAFLD.

### GENERAL DESCRIPTION OF THE INVENTION

The present invention relates to lipid-based compositions comprising antioxidants and polyphenols of natural origin, useful in the prevention and treatment of non-alcoholic fatty liver disease (NAFLD), which manage to increase insulin sensitivity, reduce inflammation of adipose tissue, reduce liver inflammation and slow liver fibrosis.

In one embodiment, a lipid-based composition comprising bioactive components, particularly antioxidants and polyphenols of natural origin.

In certain embodiments, the lipid composition of the invention contains a mixture of bioactives agents of natural origin that act directly on insulin resistance and the development of obesity, as well as on other pathogenic mechanisms of NAFLD, among which are enzyme activity, such as COX-2 cyclooxygenases, the synthesis of prostaglandins, the proliferation of cancer cells, liver damage by activation of pathways of inflammation and necrosis of the liver, adipocyte hypertrophy in white adipose tissues, liver fibrosis, lipogenesis and stress oxidative.

In some embodiments, the bioactive agents of natural origin include at least one phospholipid, a mixture of omega-3 and omega-7 fatty acids, mixtures of medium chain triglycerides (MTCs), at least one fat-soluble vitamin, and a mixture of antioxidants and polyphenols.

In a preferred embodiment, antioxidants included in the lipid-based composition of the invention comprise anthocyanins. In an even more preferred embodiment, antioxidants included in the lipid-based composition of the invention include cyanidin-3-glucoside.

In a further embodiment, the lipid-based composition of the invention comprises polyphenols of natural origin, such as curcuminoids.

Other embodiments of the invention relate to a food or nutraceutical products, dietary supplements, food supplements, or special diet foods, particularly foods for special medical purposes. In a certain modality, food or nutraceutical products, dietary supplements, food supplements, or special diet foods comprise, in addition to the lipid-based composition described above, at least one excipient selected from a group including, but not limited to, thickeners, texturizers, colorants, acidulants, and/or natural flavorings.

In one embodiment, the lipid-based composition with antioxidants and polyphenols of the invention can be consumed directly. In another embodiment, the composition of the invention can be incorporated in different matrices to be used in the food industry, including but not limited to, powdered drinks or ready-to-drink (RTD), cookies, bakery products, snacks, as well as beverages and dairy products.

In additional embodiments, the lipid composition of the invention is effective in decreasing the infiltration of lipids in the liver. In other embodiments, the lipid composition of the invention improves the anthropometric, hemodynamic and / or biochemical parameters related to NAFLD. In other embodiments, the composition of the invention improves physiopathological phenomena associated with the development of diseases related to NAFLD.

In a further embodiment, this application relates to the use of the lipid-based composition of the invention in the preparation of a medicament useful in the treatment of NAFLD or associated diseases. In other embodiments, the application relates to the use of the lipid-based composition of the invention to improve the anthropometric, hemodynamic and/or biochemical parameters related to NAFLD.

In other embodiments, the present invention relates to a method for treating or preventing NAFLD, in patients suffering from or at risk of suffering from said disease or related diseases, wherein the method comprises orally administering to the individual a therapeutically effective amount per day of a lipid-based composition of the invention.

### DETAILED DESCRIPTION OF THE INVENTION

Detailed embodiments of the present invention are described below in the present document; however, it should be understood that they are merely illustrative and can be incorporated in various forms. In addition, each of the examples given in relation to the various embodiments of the invention are intended to be illustrative and not restrictive.

The present invention relates to a lipid-based composition with bioactives of natural origin. In a preferred embodiment, the lipid-based composition comprising antioxidants and polyphenols of natural origin.

In a particular embodiment of the invention, the composition comprises a mixture of lipids of natural origin. In a preferred embodiment of the invention, the lipids of natural origin provide bioactive agents among which are at least a phospholipid, omega-3 and omega-7 fatty acids, medium chain triglycerides (MTCs), fat-soluble vitamins, antioxidants and polyphenols.

In a preferred embodiment, the lipid-based composition of the invention comprises at least one antioxidant of natural origin in a range of between about 1.0 to about 3.0 weight percent. In another embodiment, the lipid-based composition of the invention includes at least one antioxidant in a range of 2.0 to 2.5 weight percent.

In still other embodiments, the antioxidant of natural origin included in the lipid-based composition of the invention is of the anthocyanin family. In a preferred embodiment, said anthocyanin is cyanidin-3-glucoside (C3G), also called chrysanthamine.

In a particular embodiment, the anthocyanins present in the composition of the invention are obtained from dark colored fruits and vegetables. In a still more preferred embodiment, cyanidin-3-glucoside is obtained from the Chilean maqui, *Aristotelia chilensis.*

In another embodiment, the invention relates to a lipid-based composition with bioactive agents comprising at least one polyphenol of natural origin, wherein the at least one polyphenol is in a range from about 0.4 to about 1.0 weight percent. In a preferred embodiment the at least one polyphenol is between about 0.5 to about 0.8 weight percent.

In a preferred embodiment, at least one polyphenol found in the lipid-based composition of the invention is obtained from turmeric, that is, it corresponds to a curcuminoid or to a mixture thereof. In a further embodiment, the polyphenol of natural origin present in the composition of the invention comprises one or more curcuminoids selected from a group including, but not limited to, curcumin, demethoxycurcumin and bisdemethoxyurcumin.

In one embodiment, the lipid-based composition with bioactive agents of the invention includes a mixture of two or more omega-3 unsaturated fatty acids. In a preferred embodiment, the omega-3 unsaturated fatty acids are selected from a group comprising docosahexaenoic acid (DHA), Eicosapentaenoic acid (EPA), and α-linolenic acid (ALA).

In a preferred embodiment, the lipid-based composition of the invention includes a mixture of DHA and EPA. In an even more preferred embodiment, the composition of the invention comprises from about 14 to about 25% of DHA and from about 7 to about 13% of EPA. In still another preferred embodiment, the mixture comprises DHA and EPA in a ratio of 2: 1, respectively.

In a preferred embodiment, the lipid-based composition with bioactives of the invention includes from about 14 to about 23 weight percent of a phospholipid, more preferably from about 17 to about 21 weight percent.

In a particular embodiment, the lipid-based composition of the invention comprises a phospholipid selected from a group including, but not limited to, phosphatidylcholine, phosphatidylserine, sphingomyelin and / or phytosphingosine. In an even more preferred embodiment, the phospholipid is phosphatidylcholine.

In a preferred embodiment, the phospholipid is obtained from natural sources including, but not limited to, soy lecithin.

In one embodiment, the lipid-based composition with bioactives of the invention comprises a mixture of medium chain triglycerides (MCTs) in a range of between about 38 to about 55 weight percent. In other embodiments, the MTCs are in a range from about 45 to about 50 weight percent.

In one embodiment, the mixture of MTCs included in the lipid-based composition of the invention, comprises esters of glycerol fatty acids of 8 to 10 carbon atoms. In an even more preferred embodiment, the MTCs are glycerol esters of caprylic and capric acid mixtures.

In a further embodiment, the lipid-based composition with bioactives of the invention comprises at least one fat-soluble vitamin in a range from about 1.0×10⁻⁴ to about 3.0×10⁻⁴ percent by weight. In other embodiments, the composition of the invention includes at least one fat-soluble vitamin in a range from about 1.5×10⁻⁴ to about 2.5×10⁻⁴ percent by weight.

In a preferred embodiment, the lipid base composition of the invention comprises at least one fat-soluble vitamin selected from a group consisting of vitamin D3, vitamin E, and mixtures thereof.

In one embodiment, the lipid-based composition with bioactive agents of the invention comprises at least one omega-7 unsaturated fatty acid in a range from about 5 to about 15 weight percent. In another embodiment, the composition comprises at least one omega-7 unsaturated fatty acid in a range of between about 6 to about 10 weight percent.

In one embodiment, the at least one omega-7 unsaturated fatty acid comprises palmitoleic acid. In a preferred embodiment, palmitoleic acid is obtained from buckthorn oil.

In one embodiment, the lipid-based composition of the invention is in a form suitable for direct consumption, such as in the form of encapsulation, liquid emulsion, microencapsulated powder or a suspension.

In another embodiment, the lipid-based composition of the invention can be incorporated into different matrices commonly employed in the food industry, such as powdered or ready-to-eat beverages, smoothies, creams, cookies, bakery products, snacks, beverages and dairy products.

In some embodiments, the lipid-based composition of the invention is provided as a gel, dispersion, suspension or solution in oil.

In some embodiments, the use of the lipid-based composition of the invention directly influences the pathophysiological mechanisms of NAFLD. In other embodiments the use of the composition of the invention improves insulin sensitivity, reduces adipose tissue inflammation and hepatic inflammation and slows liver fibrosis.

In another embodiment the use of the lipid-based composition of the invention improves the infiltration of lipids in the liver, as do the anthropometric, hemodynamic and/or biochemical parameters related to NAFLD.

In another embodiment, the application provides a method for treating or preventing NAFLD in individuals suffering from said disease or at risk of developing it. In a preferred embodiment, the application relates to a method of treatment comprising orally administering daily a therapeutically effective amount of a composition according to any of the modalities described herein to the individual in need thereof.

In said embodiments, the effective amount of the composition is based, at least in part, on the weight of the human being who needs it.

### Examples

The following examples are intended to illustrate the invention and should not be construed as limiting the invention in any way.

### Example 1

Lipid based compositions were prepared from natural ingredients, the content of which is shown in Table 1:

**Table 1: Exemplary compositions of the invention**

| **Source** | **% in weigh** |
|---|---|
| Soy lecithin | 14-21 |
| DHA | 14-25 |
| EPA | 7-13 |
| Caprylic and capric acid triglycerides | 38-55 |
| Chilean Maqui powder | 1-3 |
| Vitamin D3 | 0.0001-0.0003 |
| Buckthorn oil | 6-10 |
| Tumeric | 0.4-0.8 |

The daily administration of the lipid-based composition comprising bioactives of natural origin has the ability to improve the physiopathological phenomena associated with the development of liver diseases such as NAFLD.

Thus, the lipid-based composition developed by the inventors offers an effective non-pharmacological alternative for the treatment of patients suffering from these diseases or at risk of developing them.

## Claims

1. A lipid-based composition comprising bioactives of natural origin, wherein said bioactive agents include an antioxidant such as cyanidin-3-glucoside, one or more polyphenols, a phospholipid, a mixture of at least two omega-3 fatty acids, a mixture of medium chain triglycerides (MCT), at least one fat-soluble vitamin, and an omega-7 fatty acid.

2. The composition of claim 1, wherein the anthocyanin is obtained from Chilean Maqui, *Aristotelia chilensis.*

3. The composition of claim 1 or 2, wherein the polyphenols comprise a mixture of curcuminoids.

4. The composition of claim 1, 2, or 3, wherein the mixture of omega-3 fatty acids comprises a mixture of DHA/EPA with a ratio of 2:1, respectively.

5. The composition of claim 1, 2, 3, or 4 wherein the phospholipid is selected from a group including phosphatidylcholine, phosphatidylserine, sphingomyelin and phytosphingosine.

6. The composition of claim 1 to 5 wherein the fat-soluble vitamin is vitamin D3, vitamin E or a mixture thereof.

7. The composition of any of claims 1 to 6, wherein the MCTs comprise glycerol esters of fatty acids of between 8 and 10 carbon atoms.

8. A food product, nutraceutical, dietary supplement, food supplement, or special diet foods, particularly foods for special medical purposes, comprising a composition according to any of claims 1 to 7.

9. A method for treating NAFLD comprising orally administering to the individual in need thereof an effective amount of a composition according to any of the modalities described herein.

10. The method of claim 9 wherein the administration of the composition is daily.

11. The use of a composition according to any of claims 1 to 7 to prevent or reduce the infiltration of lipids in the liver.

12. The use of a composition according to any of claims 1 to 7 to improve insulin sensitivity in an individual who requires it.

13. The use of a composition according to any of claims 1 to 7 to improve the anthropometric, hemodynamic and/or biochemical parameters related to NAFLD.

14. The use of a composition according to any of claims 1 to 7 for the preparation of a medicament useful in the treatment or prevention of a liver disease.

15. The use according to claim 14 wherein the liver disease is NAFLD.
